# EUROPÄISCHE PATENTSCHRIFT

(11) **EP 4 460 432 B1**
(45) Veröffentlichungstag und Bekanntmachung des Hinweises auf die Patenterteilung: **07.10.2026**
(21) Anmeldenummer: 22822598.3
(22) Anmeldetag: 01.12.2022
(51) Int. Cl.: G06F 3/01, B60K 35/00, A61M 21/02, A61M 21/00, B60Q 9/00, B60R 16/037, B60K 35/10, B60K 35/22, B60K 35/25, B60K 35/28, B60K 35/60, B60K 35/80

(54) **VERFAHREN ZUR STEIGERUNG DES NUTZERKOMFORTS EINES FAHRZEUGINSASSEN UND FAHRZEUG**
METHOD FOR INCREASING THE USER COMFORT OF A VEHICLE OCCUPANT, AND VEHICLE
PROCÉDÉ PERMETTANT D'AUGMENTER LE CONFORT DE L'UTILISATEUR D'UN OCCUPANT DE VÉHICULE ET VÉHICULE

(30) Priorität: 03.01.2022 DE 102022000029
(43) Veröffentlichungstag der Anmeldung: 13.11.2024
(73) Patentinhaber: Mercedes-Benz Group AG, 70372 Stuttgart (DE)
(72) Erfinder: SCHÖNHERR, Gudrun, 71088 Holzgerlingen (DE); MARIAN, Svetlana, 71034 Böblingen (DE)
(74) Vertreter: Novagraaf Group
(86) Internationale Anmeldenummer: PCT/EP2022/084003
(87) Internationale Veröffentlichungsnummer: WO 2023/126121

(56) Entgegenhaltungen:
- US-A1- 2016 370 863
- US-A1- 2018 027 520
- US-A1- 2020 001 895
- CHOI KYUNG YUN YUN_CHOI@MEDIA MIT EDU ET AL: "aSpire: Clippable, Mobile Pneumatic-Haptic Device for Breathing Rate Regulation via Personalizable Tactile Feedback", EXTENDED ABSTRACTS OF THE 2021 CHI CONFERENCE ON HUMAN FACTORS IN COMPUTING SYSTEMS, ACMPUB27, NEW YORK, NY, USA, 8 May 2021 (2021-05-08), pages 1 - 8, XP058554482, ISBN: 978-1-4503-8095-9, DOI: 10.1145/3411763.3451602

## Beschreibung

Die Erfindung betrifft ein Verfahren zur Steigerung des Nutzerkomforts eines Fahrzeuginsassen während eines Aufenthalts in einem Fahrzeug nach der im Oberbegriff von Anspruch 1 näher definierten Art sowie ein Fahrzeug zur Durchführung des Verfahrens.

Während der Fahrt mit einem Fahrzeug können für Fahrzeuginsassen Situationen auftreten, die zu Stress führen, wie beispielsweise das Verweilen in einem Stau. Zur Ablenkung und zur Stressreduktion können verschiedene Fahrzeugfunktionen wie Radio hören, Musik streamen, das Spielen auf einer Videospielkonsole oder das Schauen von Videos über ein Infotainmentsystem des Fahrzeugs genutzt werden. Einige Fahrzeuge bieten auch die Funktion zur Durchführung von Entspannungsübungen, beispielsweise Atemübungen, an. Dabei werden die Fahrzeuginsassen vom Fahrzeug zur Durchführung der Entspannungsübung angeleitet. Hierzu kann das Fahrzeug visuelle Informationen über eine Anzeige ausgeben und/oder akustische Informationen über Lautsprecher ausgeben. Die Durchführung der Entspannungsübung kann durch das Abspielen entspannender Musik unterstützt werden. Zur Durchführung einer solchen Entspannungsübung muss der Fahrzeuginsasse jedoch das entsprechende Entspannungsprogramm zuerst manuell starten, was für den Fahrzeuginsassen mit Aufwand verbunden ist. Dies führt häufig dazu, dass solche Entspannungsfunktionen nur selten genutzt werden.

Ferner sind sogenannte Wearables wie smarte Armbänder oder Smartwatches bekannt, die den Vitalzustand des Trägers überwachen und Empfehlungen zur Durchführung von Entspannungsübungen nach Bedarf ausgeben. Dabei wird der Träger durch das Wearable zur genauen Durchführung der Entspannungsübung angeleitet. So gibt das Wearable beispielsweise mittels eines Aktuators ein haptisches Feedback aus, was bei der Durchführung einer Atemübung dazu veranlassen soll, gezielt Ein- und Auszuatmen. Das haptische Feedback ist dabei an die Atemfrequenz angepasst, sodass der Träger genau dann Ein- und Ausatmen soll, wenn ein bestimmtes haptisches Feedback über das Wearable ausgegeben wird bzw. ausbleibt. Nachteilig ist dabei jedoch, dass ein über ein Wearable ausgegebenes haptisches Feedback oftmals nicht ausreichend von einem Träger wahrgenommen werden kann, beispielsweise weil der Träger das Wearable zu locker trägt. Insbesondere bei der Bedienung eines Fahrzeugs kann das Wearable am Handgelenk durch die Nutzung des Lenkrads verrutschen, was den Einsatz von Wearables zur Ausgabe von haptischem Feedback im Fahrzeug unpraktisch macht.

Trainingsprogramme zur Durchführung von Entspannungsübungen können auch in Form sogenannter Apps auf einem Smartphone oder Tabletcomputer geladen werden. Dabei kann das entsprechende mobile Endgerät auch mit einem passenden Wearable gekoppelt werden, sodass das Wearable Steuerungsbefehle zur Ausgabe des haptischen Feedbacks vom mobilen Endgerät empfängt.

Ferner sind Fahrzeugsitze mit integrierten Aktoren bekannt, die beispielsweise zur Massage von Fahrzeuginsassen verwendet werden können.

Zudem offenbart die US 2016/0107570 A1 ein System und ein Verfahren zur Verbesserung der kontinuierlichen Aufmerksamkeit in Fahrzeugen durch die Ausgabe haptischen Feedbacks. Das System umfasst wenigstens einen Sensor, der eine Umgebung und/oder einen Zustand eines Fahrzeugs und/oder eines Fahrzeuginsassen überwacht. Hierdurch können beispielsweise benachbarte Verkehrsteilnehmer sowie deren Relativposition gegenüber dem Fahrzeug erkannt werden und/oder ein Gesundheits- und/oder Aufmerksamkeitszustand von Fahrzeuginsassen erfasst werden. Das Fahrzeug kann einen oder mehrere Aktoren umfassen, die haptisches Feedback ausgeben. Diese können beispielsweise im Fahrzeugsitz, im Lenkrad oder in einen Gurt integriert sein. Auch können Wearables zur Ausgabe von haptischem Feedback genutzt werden. Das System gibt in Abhängigkeit der vom Sensor erfassten Größen haptisches Feedback an einen Fahrzeuginsassen weiter. So kann dem Fahrer beispielsweise über haptisches Feedback mitgeteilt werden, dass sich ein weiteres Fahrzeug in einem toten Winkel befindet oder dass der Fahrer gerade eine Fahrspurmarkierung überfährt. Das haptische Feedback kann auch Richtungsinformationen umfassen und beispielsweise eine in eine bestimmte Orientierung gerichtete Bewegung aufweisen. Über das haptische Feedback kann der Fahrer auch "angestoßen" werden, was seine Aufmerksamkeit verbessert.

Ferner offenbart die US 2016/370863 A1 ein Gerät zur Richtungs- und Aufmerksamkeitsanweisung. Das Gerät kann Aktoren zum Aufprägen eines haptischen Feedbacks an einen Nutzer ansteuern. Entsprechende Steuersignale werden vom Gerät von einem mobilen Endgerät wie einem Smartphone empfangen. Die Aktoren zum Aufprägen des haptischen Feedbacks werden dabei von einem Nutzer am Körper getragen oder sind in einen Sitz, wie einen Fahrzeugsitz, integriert.

Ferner offenbart die US 2018/027520 A1 eine haptische Funktion für über ein Netzwerk verbundene Geräte. Ein Signal zum Ausgeben von haptischen Feedback wird von einem ersten Gerät an ein zweites Gerät übertragen, wobei das zweite Gerät einen entsprechenden Aktor zur Erzeugung des haptischen Feedbacks ansteuert.

Ferner offenbart: CHOI KYUNG YUN YUN_CHOI@MEDIA MIT EDU ET AL: "aSpire: Clippable, Mobile Pneumatic-Haptic Device for Breathing Rate Regulation via Personalizable Tactile Feedback", EXTENDED ABSTRACTS OF THE 2021 CHI CONFERENCE ON HUMAN FACTORS IN COMPUTING SYSTEMS, ACMPUB27, NEW YORK, NY, USA, 8. Mai 2021 (2021-05-08), Seiten 1-8, XP058554482,
DOI: 10.1145/3411763.3451602
ISBN: 978-1-4503-8095-9
ein an einem Sicherheitsgurt eines Fahrzeugs montierbares Gerät zum Aufprägen eines haptischen Feedbacks an einen Nutzer mittels aufblasbarer Luftkissen. Das Gerät dient dazu, die Atemfrequenz des Nutzers zu regulieren.

Zudem offenbart die US 2020/001895 A1 ein ADAS System, nutzend haptische Stimuli die durch ein Wearable generiert werden. Dabei wird ein haptisches Feedback an einen das Wearable tragenden Nutzer ausgegeben, wenn das ADAS System ein Signal ausgibt.

Der vorliegenden Erfindung liegt die Aufgabe zugrunde, ein Verfahren zur Steigerung des Nutzerkomforts eines Fahrzeuginsassen während eines Aufenthalts in einem Fahrzeug anzugeben, mit dessen Hilfe der Nutzerkomfort besonders zuverlässig und komfortabel gesteigert werden kann.

Erfindungsgemäß wird diese Aufgabe durch ein Verfahren zur Steigerung des Nutzerkomforts eines Fahrzeuginsassen mit den Merkmalen des Anspruchs 1 gelöst. Vorteilhafte Ausgestaltungen und Weiterbildungen sowie ein Fahrzeug, in dem das erfindungsgemäße Verfahren zur Anwendung kommt, ergeben sich aus den hiervon abhängigen Ansprüchen.

Bei einem Verfahren zur Steigerung des Nutzerkomforts eines Fahrzeuginsassen während eines Aufenthalts in einem Fahrzeug der eingangs genannten Art wird dem Fahrzeuginsassen mittels wenigstens eines im Fahrzeug angeordneten Aktors ein haptisches Feedback vermittelt, wobei der Aktor durch eine Recheneinheit des Fahrzeugs in Abhängigkeit von einem mobilen Endgerät empfangener Informationen angesteuert wird, wobei:
- auf einem ersten mobilen Endgerät ein Programm ausgeführt wird, welches Steuersignale zum Betätigen eines haptischen Feedback-Aktors des ersten mobilen Endgeräts ausgibt;
- das erste mobile Endgerät unmittelbar mit der Recheneinheit des Fahrzeugs oder mittelbar über ein zweites mobiles Endgerät mit der Recheneinheit des Fahrzeugs kommuniziert, wobei die vom auf dem ersten mobilen Endgerät ausgeführten Programm ausgegebenen Steuersignale an die Recheneinheit übermittelt werden; und
- die Recheneinheit des Fahrzeugs den wenigstens einen im Fahrzeug angeordneten Aktor zur Erzeugung des haptischen Feedbacks in Abhängigkeit der vom ersten mobilen Endgerät empfangenen Steuersignale ansteuert.

Erfindungsgemäß initiiert dabei das auf dem ersten mobilen Endgerät ausgeführte Programm die Ausgabe von Steuersignalen in Abhängigkeit einer Anleitung zur Durchführung von Atemübungen, wobei mehrere Fahrzeuginsassen gleichzeitig ein Atemtraining durchführen, wobei die Atemübung individuell auf die unterschiedlichen Fahrzeuginsassen angepasst wird, sodass jeder Fahrzeuginsasse ein individuelles Training absolviert.

Bei dem ersten mobilen Endgerät kann es sich um ein Wearable wie ein smartes Bracelet oder eine Smartwatch handeln oder auch um einen Tabletcomputer oder ein Smartphone. Auf einem solchen mobilen Endgerät lassen sich Programme, auch als Apps bezeichnet, ausführen, die das Ausgeben eines haptischen Feedbacks über das jeweilige mobile Endgerät bewirken. Diese Steuersignale lassen sich von der Recheneinheit des Fahrzeugs abgreifen und zum Ansteuern eines im Fahrzeug verbauten Aktors zur Erzeugung haptischen Feedbacks nutzen. Mit anderen Worten steuert das erste mobile Endgerät das über das Fahrzeug ausgegebene haptische Feedback fern. Dies hat entscheidende Vorteile für einen Fahrzeuginsassen. So ist ein über im Fahrzeug angeordnete Aktoren ausgegebenes haptisches Feedback deutlicher vom Fahrzeuginsassen wahrnehmbar, als ein über ein Wearable oder Smartphone ausgegebenes haptisches Feedback. Zudem erfolgt die Ausgabe des haptischen Feedbacks automatisch, veranlasst durch das auf dem ersten mobilen Endgerät ausgeführten Programms. Hierdurch lassen sich Fahrzeuginsassen komfortabel an bestimmte Dinge erinnern oder zur Durchführung bestimmter Tätigkeiten veranlassen.

Handelt es sich bei dem ersten mobilen Endgerät um ein Wearable, so kann das Programm, also die App, auch auf einem zweiten mobilen Endgerät ausgeführt werden. In diesem Fall handelt es sich bei dem zweiten mobilen Endgerät um einen Tabletcomputer oder um ein Smartphone. Das erste mobile Endgerät und das zweite mobile Endgerät sind dann miteinander gekoppelt, was eine Kommunikation eines Wearables mit der Recheneinheit des Fahrzeugs ermöglicht, da die Recheneinheit des Fahrzeugs ebenfalls mit dem Tabletcomputer oder Smartphone gekoppelt ist.

Zum Koppeln der mobilen Endgeräte untereinander sowie mit der Recheneinheit des Fahrzeugs kann eine beliebige digitale Kommunikationstechnik verwendet werden wie kabelgebundene oder drahtlose Kommunikationstechnologien. Beispielsweise kann ein Tabletcomputer oder Smartphone mit einem USB-Kabel an die Recheneinheit angeschlossen werden. Zur Kommunikation eines Wearables mit einem Smartphone oder Tabletcomputer sowie zur Kommunikation eines Tabletcomputers oder Smartphones mit der Recheneinheit kann beispielsweise Bluetooth, WIFI, NFC Ultrabreitbandkommunikation oder dergleichen eingesetzt werden.

Die Ausgabe des haptischen Feedbacks im Fahrzeug kann sowohl während der Fahrt mit dem Fahrzeug als auch im Stand erfolgen. Die entsprechenden Aktoren können im Fahrzeug an einer beliebigen Stelle angeordnet sein, was das Vermitteln des haptischen Feedbacks an die Fahrzeuginsassen ermöglicht, solange ein entsprechender Fahrzeuginsasse eine entsprechende Stelle, an der ein entsprechender Aktor angeordnet ist, zu gegebenen Zeitfenstern berührt. Beispielsweise kann ein oder können mehrere Aktoren in einen Fahrzeugsitz, in das Lenkrad des Fahrzeugs, in Bedienelemente, in einen Gurt oder dergleichen integriert sein. Als Aktoren können jegliche bewährte Aktoren zur Erzeugung eines haptischen Feedbacks wie Elektroantriebe mit Exzentermasse, Piezoelemente und dergleichen verwendet werden. Das haptische Feedback weist eine Modulation auf. Dies bedeutet, dass es in seiner Stärke, Frequenz, Dauer und dergleichen variabel ist. Das im Fahrzeug ausgegebene haptische Feedback kann der Modulation des ursprünglich zur Ausgabe über den haptischen Feedback-Aktor des ersten mobilen Endgeräts vorgesehenen haptischen Feedbacks entsprechen.

Das haptische Feedback kann an eine fahrzeugführende Person, einen Beifahrer und/oder auch an Fahrgäste im Fond ausgegeben werden. Den Fahrzeuginsassen kann dabei das gleiche oder ein individuelles haptisches Feedback aufgeprägt werden.

Wie bereits beschrieben, sieht das Verfahren vor, dass das auf dem ersten mobilen Endgerät ausgeführte Programm die Ausgabe von Steuersignalen in Abhängigkeit einer Anleitung zur Durchführung von Atemübungen initiiert. Über die Kopplung des Smartphones mit der Recheneinheit des Fahrzeugs lassen sich dann auch die im Fahrzeug angebrachten Aktoren betätigen.

In der erfindungsgemäßen Ausgestaltung des erfindungsgemäßen Verfahrens handelt es sich bei dem Programm um ein Programm zur Durchführung von Entspannungsübungen in Form von Atemübungen. Ein solches Programm leitet eine Person dazu an, sich auf ihre Atmung zu fokussieren und somit gezielt ein- und auszuatmen, beziehungsweise die Luft anzuhalten. Beispielsweise kann ein Atemmuster vorsehen, dass die Person vier Sekunden einatmen, sieben Sekunden lang ihren Atem anhalten und acht Sekunden lang ausatmen soll, oder, wie beim sogenannten "Box Breathing" jeweils vier Sekunden ein- und ausatmen soll, mit jeweiligen Atempausen von vier Sekunden dazwischen.

Diese Atemtechniken werden typischerweise durch visuelle Informationen auf einer Anzeige des Smartphones, Tabletcomputers und/oder Wearables ergänzt. Analog wird ein zum Einatmen, Luft anhalten und/oder Ausatmen passendes haptisches Feedback über das Wearable ausgegeben. Beispielsweise kann ein anschwellendes haptisches Feedback zur Symbolisierung eines Einatemvorgangs, ausbleibendes haptisches Feedback zur Symbolisierung von Luft anhalten und ein abschwellendes haptisches Feedback zur Symbolisierung eines Ausatemvorgangs vermittelt werden. Durch die Ausgabe des haptischen Feedbacks über das Fahrzeug lässt sich dieses von den Fahrzeuginsassen ganz besonders deutlich und komfortabel wahrnehmen.

Mit Hilfe des erfindungsgemäßen Verfahrens sind zudem mehrere Fahrzeuginsassen gleichzeitig dazu in der Lage, ein Atemtraining durchzuführen. Dabei reicht es aus, wenn ein einziger Fahrzeuginsasse ein entsprechendes erstes mobiles Endgerät mit sich führt. Das Fahrzeug, sprich die Recheneinheit, kann dann mehrere Aktoren im Fahrzeug gleichzeitig ansteuern, sodass mehrere Personen gleichzeitig an der Atemübung teilnehmen können. Generell ist es dabei auch denkbar, dass die Atemübung individuell auf die unterschiedlichen Fahrzeuginsassen angepasst wird, sodass jeder Fahrzeuginsasse ein individuelles Training absolviert.

Auch kann das haptische Feedback im Fahrzeug ausgegeben werden, um einen Fahrzeuginsassen "anzustoßen", wodurch der Fahrzeuginsasse dazu veranlasst wird eine Atemübung durchzuführen. Daraufhin kann die Anleitung zur Durchführung der Atemübung visuell und/oder akustisch erfolgen und der oder die Aktoren im Fahrzeug dazu genutzt werden das haptische Feedback in Form eines Massageprogramms während der Atemübung auszugeben.

Entsprechend einer vorteilhaften Ausgestaltung des erfindungsgemäßen Verfahrens wird das im Fahrzeug ausgegebene haptische Feedback gegenüber dem zur Ausgabe über das erste mobile Endgerät vorgesehenen haptischen Feedback in seiner Modulation angepasst. Wie bereits erwähnt kann das im Fahrzeug ausgegebene haptische Feedback dem über das erste mobile Endgerät ausgegebenen haptischen Feedback entsprechen. Es kann sich jedoch auch hierzu unterscheiden. Die Möglichkeiten zur Ausgabe des haptischen Feedbacks über das erste mobile Endgerät sind beschränkt, weil typischerweise ein mobiles Endgerät lediglich einen Aktor zur Erzeugung haptischen Feedbacks aufweist. Im Fahrzeug können jedoch mehrere Aktoren, insbesondere an verschiedenen Positionen, angeordnet sein, wodurch ein besonders umfassendes und aufregendes haptisches Feedbackerlebnis im Fahrzeug erzeugt werden kann. Während beispielsweise über eine Smartwatch beim Durchführen einer Atemübung lediglich eine Vibration am Handgelenk des Fahrzeuginsassen ausgegeben werden kann, kann beispielsweise über in einem Fahrzeugsitz integrierte Aktoren einem haptischen Feedback auch eine Richtung aufgeprägt werden. Beispielsweise kann das haptische Feedback in der Rückenlehne eines Fahrzeugsitzes nach oben wandern, um ein Einatmen zu symbolisieren und in der Rückenlehne nach unten wandern, um ein Ausatmen zu symbolisieren. Generell kann das im Fahrzeug ausgegebene haptische Feedback beispielsweise in seiner Frequenz, Dauer, Stärke, Richtung und dergleichen zum ursprünglich über das erste mobile Endgerät ausgegebenen haptischen Feedback unterschiedlich ausgeführt sein.

Entsprechend einer weiteren vorteilhaften Ausgestaltung des Verfahrens werden zusätzlich zum im Fahrzeug ausgegebenen haptischen Feedback, vom Kontext der vom ersten mobilen Endgerät empfangenen Steuersignale abhängige Informationen auf einer Anzeige im Fahrzeug visuell dargestellt. Erhält der Fahrzeuginsasse beispielsweise einen Anruf, so können Kontaktinformationen sowie eine Schaltfläche zum Anruf annehmen oder Anruf ablehnen auf einer Anzeige im Fahrzeug dargestellt werden. Analog lässt sich die Uhrzeit, eine Stoppuhr oder ein Countdown-Timer auf der Anzeige darstellen. Ebenfalls lassen sich die auf dem Display des Smartphones bzw. Tabletcomputers dargestellten Anleitungen beziehungsweise Informationen zur Durchführung einer Atemübung auf das Display des Fahrzeugs übertragen. Eine solche Anleitung umfasst oftmals eine Animation, wie ein an- und abschwellendes geometrisches Objekt.

Eine weitere vorteilhafte Ausgestaltung des Verfahrens sieht es ferner vor, dass der Fahrzeuginsasse die Art und Weise, wie das haptische Feedback im Fahrzeug ausgegeben wird und/oder wie die Informationen im Fahrzeug visuell dargestellt werden, einstellt. Hierdurch kann der Fahrzeuginsasse seinen persönlichen Vorlieben gerecht werden. Beispielsweise kann der Fahrzeuginsasse einstellen, dass sich das im Fahrzeug ausgegebene haptische Feedback von dem über das Wearable oder das Smartphone ausgegebenen haptischen Feedbacks unterscheiden soll. Dabei kann er auch festlegen, wie eine unterschiedliche Modulation des im Fahrzeug ausgegebenen haptischen Feedbacks genau aussehen soll. Auch kann der Fahrzeuginsasse die Darstellung der Informationen auf der Anzeige im Fahrzeug an seine Vorlieben anpassen. Beispielsweise kann er auswählen, ob eine Information als Text und/oder graphisch dargestellt werden soll und beispielsweise eine Position einer entsprechenden Grafik auf der Anzeige festlegen.

Bevorzugt gibt, wenn die Recheneinheit des Fahrzeugs Steuersignale vom ersten mobilen Endgerät empfängt, die Recheneinheit eine Aufforderung zur Eingabe einer bestätigenden Bedienhandlung an den Fahrzeuginsassen aus und gibt das haptische Feedback im Fahrzeug erst dann aus, wenn der Fahrzeuginsasse die bestätigende Bedienhandlung eingibt. So verfügen Smartwatches oder smarte Armbänder oftmals über eine Erinnerungsfunktion, dass nach Ablaufen einer festgelegten Zeit, beispielsweise einmal pro Stunde, eine bestimmte Aktivität durchgeführt werden soll. Solche Erinnerungen sehen beispielsweise das Aufstehen und Herumlaufen, das Abwenden von einer Bildschirmtätigkeit, das Durchführen einer Atemübung oder dergleichen vor. Dabei kann die Erinnerung durch die Ausgabe haptischen Feedbacks unterstützt werden. Während der Fahrt ist eine fahrzeugführende Person oftmals auf das Fahrgeschehen fokussiert. Dabei besteht also leicht die Möglichkeit, dass die fahrzeugführende Person gestresst wird und vergisst, eine entspannte Haltung einzunehmen. Beispielsweise kann eine solche Erinnerung eine Aufforderung zur Durchführung einer Atemübung aufweisen. So kann die Recheneinheit des Fahrzeugs nach Empfangen einer solchen Erinnerung den Fahrzeuginsassen fragen, ob er eine entsprechende Atemübung durchführen möchte. Somit lässt sich die Häufigkeit, mit der ungenutzte Fahrzeugfunktionen genutzt werden, erhöhen. Die Atemübung kann dabei der auf dem ersten mobilen Endgerät ausführbaren Atemübung entsprechen, oder die fahrzeugführende Person bzw. der Fahrzeuginsasse kann eine von mehreren durch die Recheneinheit des Fahrzeugs bereitgestellte Atemübungen auswählen. Generell ist es dabei natürlich auch möglich, dass die Atemübung direkt bei Empfangen der Erinnerung zur Durchführung der Entspannungstechnik automatisch gestartet wird. Ansonsten kann zur Eingabe einer entsprechenden Bedienhandlung beispielsweise ein Touchdisplay im Fahrzeug genutzt werden oder eine Eingabe mittels Sprachbefehle erfolgen.

Bei einem Fahrzeug mit einer Recheneinheit und einer Kommunikationsschnittstelle sind erfindungsgemäß die Recheneinheit und die Kommunikationsschnittstelle zur Durchführung eines im vorigen beschriebenen Verfahrens eingerichtet. Bei dem Fahrzeug kann es sich um ein beliebiges Fahrzeug wie einen Pkw, Lkw, Transporter, Bus oder dergleichen handeln. Bei der Recheneinheit kann es sich um einen zentralen Bordcomputer, eine Telematikeinheit oder ein sonstiges Steuergerät eines Fahrzeuguntersystems handeln. Mit Hilfe der Kommunikationsschnittstelle kann die Recheneinheit eine Kommunikationsverbindung zum ersten mobilen Endgerät und/oder dem zweiten mobilen Endgerät aufbauen. Ebenfalls kann die Recheneinheit über die Kommunikationsschnittstelle, beispielsweise per Mobilfunk, eine Kommunikationsverbindung zu einer zentralen Recheneinheit, sprich einem Cloudserver, aufbauen. Über den Cloudserver können beispielsweise neue Atemübungen und/oder verschiedene, sich durch ihre Modulation unterscheidende, haptische Feedbacks abgerufen werden.

Weitere vorteilhafte Ausgestaltungen des erfindungsgemäßen Verfahrens zur Steigerung des Nutzerkomforts eines Fahrzeuginsassen ergeben sich auch aus den Ausführungsbeispielen, welche nachfolgend unter Bezugnahme auf die Figuren näher beschrieben werden.

Dabei zeigen:
- Fig. 1: eine schematische Ansicht der zur Durchführung eines erfindungsgemäßen Verfahrens zur Steigerung des Nutzerkomforts eines Fahrzeuginsassen erforderlichen Komponenten; und
- Fig. 2: ein schematisiertes Ablaufdiagramm eines erfindungsgemäßen Verfahrens.

Während der Fahrt mit einem Fahrzeug können für Fahrzeuginsassen stressige Situationen auftreten. Zur Reduktion eines Stresslevels eines jeweiligen Fahrzeuginsassen können Entspannungstechniken wie Atemübungen durchgeführt werden. Mit Hilfe eines erfindungsgemäßen Verfahrens wird die Durchführung von Atemübungen im Fahrzeug durch die Ausgabe von haptischem Feedback im Fahrzeug unterstützt, wodurch sich der Nutzerkomfort für die Fahrzeuginsassen während des Aufenthalts im Fahrzeug verbessern lässt. Das im Fahrzeug ausgegebene haptische Feedback kann gemäß einer nicht unter den Schutzumgang der Erfindung zählenden Ausführungsform außerdem zur Identifikation eingehender Anrufer sowie zur Veranschaulichung verstrichener Zeit eingesetzt werden.

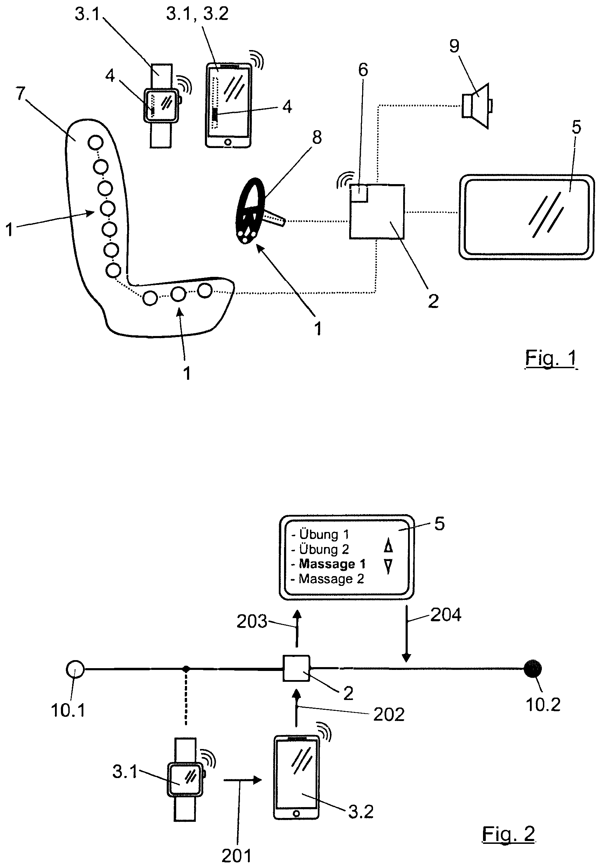

Die zur Durchführung eines erfindungsgemäßen Verfahrens verwendeten Komponenten sind in Figur 1 dargestellt. Das haptische Feedback wird über einen oder mehrere im Fahrzeug angeordnete Aktoren 1 ausgegeben. Beispielsweise können ein oder mehrere Aktoren 1 in einen Fahrzeugsitz 7 und/oder das Lenkrad 8 des Fahrzeugs integriert sein. Angesteuert werden der oder die Aktoren 1 durch eine Recheneinheit 2, welche ihrerseits über eine Kommunikationsschnittstelle 6 Steuerungsbefehle von einem ersten mobilen Endgerät 3.1 empfängt. Das erste mobile Endgerät 3.1 kann unmittelbar mit der Recheneinheit 2 kommunizieren oder mittelbar über ein zweites mobiles Endgerät 3.2.

Auf dem ersten mobilen Endgerät 3.1 wird ein Programm zur Anleitung einer Entspannungstechnik in Form einer Atemübung ausgeführt. Das Programm gibt Steuerungsbefehle zur Ausgabe eines haptischen Feedbacks über einen in das erste mobile Endgerät 3.1 integrierten haptischen Feedback-Aktor 4 aus. Die entsprechenden Steuersignale werden von der Recheneinheit 2 abgegriffen und zum Aufprägen des haptischen Feedbacks an die Aktoren 1 verwendet.

Die Ausgabe des haptischen Feedbacks im Fahrzeug kann durch visuelle Informationen über eine Anzeige 5 und/oder akustische Informationen über einen Lautsprecher 9 im Fahrzeug ergänzt werden. Über den Lautsprecher 9 kann beispielsweise eine akustische Anleitung zur Durchführung einer Atemübung ausgegeben werden und/oder eine Atemübung durch die Ausgabe von entspannenden Klängen noch angenehmer gestaltet werden.

Figur 2 zeigt einen schematisierten Ablauf eines erfindungsgemäßen Verfahrens. Während der Fahrt des Fahrzeugs von einem Startpunkt 10.1 zu einem Zielpunkt 10.2 registriert das erste mobile Endgerät 3.1, hier in Form einer Smartwatch, dass für einen Fahrzeuginsassen die Durchführung einer Entspannungsübung erforderlich ist. Beispielsweise kann die Smartwatch dies feststellen, indem sie das Verstreichen einer festgelegten Zeitspanne, beispielsweise eine Stunde, feststellt oder durch Auswertung von Vitalzeichen ein erhöhtes Stresslevel des Fahrzeuginsassen ermittelt. Die Smartwatch gibt dann die Empfehlung zur Durchführung der Entspannungstechnik an ein zweites mobiles Endgerät 3.2, hier in Form eines Smartphones, beispielsweise per Bluetooth wie angedeutet durch einen Pfeil 201, weiter.

Das Smartphone wiederum gibt diese Information an die Recheneinheit 2 des Fahrzeugs, wie angedeutet durch einen Pfeil 202, weiter.

Daraufhin initiiert die Recheneinheit 2, angedeutet durch einen Pfeil 203, das Ausgeben einer Aufforderung an den Fahrzeuginsassen über die Anzeige 5 des Fahrzeugs, dass das Durchführen einer Entspannungstechnik empfohlen wird. Über die Anzeige 5, beispielsweise ausgeführt als Touchscreen, kann der Fahrzeuginsasse wählen, wie nun weiter vorgegangen werden soll. So kann der Fahrzeuginsasse über die Anzeige 5 auswählen, ob die Atemübung gemäß des Vorschlags der Smartwatch ausgeführt werden soll, wobei die Aktoren 1 des Fahrzeugs analog zum über die Smartwatch ausgegebenen haptischen Feedback angesteuert werden sollen, oder ob die Aktoren 1 im Fahrzeug gemäß einer abweichenden Modulation angesteuert werden sollen. Auch kann der Fahrzeuginsasse auswählen, dass die Atemübung selbst ausschließlich durch die Smartwatch angeleitet wird, und im Fahrzeug selbst die Atemübung durch eine Massage ergänzt wird. Dabei kann der Fahrzeuginsasse auf der Anzeige 5 eins von verschiedenen Massage- oder Workoutprogrammen auswählen, bei denen jeweils die Aktoren 1 im Fahrzeug unterschiedlich angesteuert werden. Auch kann der Fahrzeuginsasse auswählen, ob die Darstellung von anleitenden oder hilfreichen Informationen auf der Anzeige 5 und/oder die Ausgabe von akustischen Anleitungen und/oder entspannenden Klängen über den Lautsprecher 9 erfolgen soll. Dabei kann der Fahrzeuginsasse auch festlegen, dass bei erneutem Empfangen einer Erinnerung zur Durchführung einer Entspannungstechnik von der Smartwatch die entsprechende von ihm getroffene Auswahl vom Fahrzeug nächstes Mal automatisch ausgewählt wird. Nun wird die entsprechende Atemübung durchgeführt und endet, angedeutet durch einen Pfeil 204.

Dank der durch die Smartwatch ausgegebenen Erinnerung bzw. dank der Überwachung des Stresslevels des Fahrzeuginsassen wird der Fahrzeuginsasse zum richtigen Moment dazu aufgefordert, eine Entspannungstechnik anzuwenden. Durch die Ausgabe des haptischen Feedbacks im Fahrzeug sowie gegebenenfalls ergänzt durch die auf der Anzeige 5 und/oder dem Lautsprecher 9 ausgegebenen Informationen lässt sich die entsprechende Entspannungstechnik im Fahrzeug ganz besonders komfortabel durchführen. Dies erhöht den Nutzerkomfort des Fahrzeuginsassen beträchtlich.

## Patentansprüche

1. Verfahren zur Steigerung des Nutzerkomforts eines Fahrzeuginsassen während eines Aufenthalts in einem Fahrzeug, wobei dem Fahrzeuginsassen mittels wenigstens eines im Fahrzeug angeordneten Aktors (1) ein haptisches Feedback vermittelt wird, und wobei der Aktor (1) durch eine Recheneinheit (2) des Fahrzeugs in Abhängigkeit von einem mobilen Endgerät (3.1, 3.2) empfangener Informationen angesteuert wird,
wobei
- auf einem ersten mobilen Endgerät (3.1) ein Programm ausgeführt wird, welches Steuersignale zum Betätigen eines haptischen Feedback-Aktors (4) des ersten mobilen Endgeräts (3.1) ausgibt;
- das erste mobile Endgerät (3.1) unmittelbar oder mittelbar über ein zweites mobiles Endgerät (3.2) mit der Recheneinheit (2) des Fahrzeugs kommuniziert, wobei die vom auf dem ersten mobilen Endgerät (3.1) ausgeführten Programm ausgegebenen Steuersignale an die Recheneinheit (2) übermittelt werden; und
- die Recheneinheit (2) des Fahrzeugs den wenigstens einen im Fahrzeug angeordneten Aktor (1) zur Erzeugung des haptischen Feedbacks in Abhängigkeit der vom ersten mobilen Endgerät (3.1) empfangenen Steuersignale ansteuert;
wobei das auf dem ersten mobilen Endgerät (3.1) ausgeführte Programm die Ausgabe von Steuersignalen in Abhängigkeit einer Anleitung zur Durchführung von Atemübungen initiiert, wobei mehrere Fahrzeuginsassen gleichzeitig ein Atemtraining durchführen, wobei die Atemübung individuell auf die unterschiedlichen Fahrzeuginsassen angepasst wird, sodass jeder Fahrzeuginsasse ein individuelles Training absolviert.

2. Verfahren nach Anspruch 1 oder 2,
wobei das im Fahrzeug ausgegebene haptische Feedback gegenüber dem zur Ausgabe über das erste mobile Endgerät (3.1) vorgesehenen haptischen Feedback in seiner Modulation angepasst wird.

3. Verfahren nach Anspruch 1 oder 2,
wobei zusätzlich zum im Fahrzeug ausgegebenen haptischen Feedback, vom Kontext der vom ersten mobilen Endgerät (3.1) empfangenen Steuersignale abhängige Informationen auf einer Anzeige (5) im Fahrzeug visuell dargestellt werden.

4. Verfahren nach einem der Ansprüche 1 bis 3,
wobei der Fahrzeuginsasse die Art und Weise wie das haptische Feedback im Fahrzeug ausgegeben wird einstellt.

5. Verfahren nach Anspruch 3 oder nach Anspruch 3 und 4,
wobei der Fahrzeuginsasse die Art und Weise wie die Informationen im Fahrzeug visuell dargestellt werden einstellt.

6. Verfahren nach einem der Ansprüche 1 bis 5,
wobei wenn die Recheneinheit (2) des Fahrzeugs Steuersignale vom ersten mobilen Endgerät (3.1) empfängt, die Recheneinheit (2) eine Aufforderung zur Eingabe einer bestätigenden Bedienhandlung an den Fahrzeuginsassen ausgibt und das haptische Feedback im Fahrzeug erst dann ausgibt, wenn der Fahrzeuginsasse die bestätigende Bedienhandlung eingibt.

7. Fahrzeug mit einer Recheneinheit (2) und einer Kommunikationsschnittstelle (6),
wobei die Recheneinheit (2) und die Kommunikationsschnittstelle (6) zur Durchführung eines Verfahrens nach einem der Ansprüche 1 bis 6 eingerichtet sind.

## Claims

1. Method for increasing the user comfort of a vehicle occupant during a stay in a vehicle, wherein the vehicle occupant is provided with haptic feedback by means of at least one actuator (1) arranged in the vehicle, and wherein the actuator (1) is controlled by a computing unit (2) of the vehicle on the basis of information received from a mobile terminal (3.1, 3.2),
wherein
- a program is executed on a first mobile terminal (3.1) and outputs control signals to actuate a haptic feedback actuator (4) of the first mobile terminal (3.1);
- the first mobile terminal (3.1) communicates directly or indirectly with the computing unit (2) of the vehicle via a second mobile terminal (3.2), wherein the control signals output by the program executed on the first mobile terminal (3.1) are transmitted to the computing unit (2); and
- the computing unit (2) of the vehicle controls the at least one actuator (1) arranged in the vehicle to generate the haptic feedback on the basis of the control signals received from the first mobile terminal (3.1);
wherein
the program executed on the first mobile terminal (3.1) initiates the output of control signals on the basis of instructions for performing breathing exercises, wherein
multiple vehicle occupants perform breathing training simultaneously, wherein the breathing exercise is individually adapted to the different vehicle occupants so that each vehicle occupant completes individual training.

2. Method according to claim 1 or 2,
wherein
the haptic feedback output in the vehicle is adapted in its modulation with respect to the haptic feedback intended for output via the first mobile terminal (3.1).

3. Method according to claim 1 or 2,
wherein,
in addition to the haptic feedback output in the vehicle, information dependent on the context of the control signals received from the first mobile terminal (3.1) is visually displayed on a screen (5) in the vehicle.

4. Method according to any of claims 1 to 3,
wherein
the vehicle occupant sets the way in which haptic feedback is output in the vehicle.

5. Method according to claim 3 or according to claim 3 and 4,
wherein
the vehicle occupant sets the way in which information is visually displayed in the vehicle.

6. Method according to any of claims 1 to 5,
wherein,
when the computing unit (2) of the vehicle receives control signals from the first mobile terminal (3.1), the computing unit (2) outputs a request to the vehicle occupant to input a confirming operating action and only outputs the haptic feedback in the vehicle when the vehicle occupant inputs the confirming operating action.

7. Vehicle having a computing unit (2) and a communication interface (6),
wherein
the computing unit (2) and the communication interface (6) are designed to perform a method according to any of claims 1 to 6.

## Revendications

1. Procédé permettant d'améliorer le confort d'un passager pendant son séjour à bord d'un véhicule, dans lequel un retour haptique est transmis au passager à l'aide d'au moins un actionneur (1) disposé dans le véhicule, et dans lequel l'actionneur (1) est commandé par une unité de calcul (2) du véhicule en fonction d'informations reçues par un terminal mobile (3.1, 3.2), dans lequel
- un programme est exécuté sur un premier terminal mobile (3.1), lequel programme émet des signaux de commande pour l'activation d'un actionneur de retour haptique (4) du premier terminal mobile (3.1) ;
- le premier terminal mobile (3.1) communique, directement ou indirectement par l'intermédiaire d'un second terminal mobile (3.2), avec l'unité de calcul (2) du véhicule, dans lequel les signaux de commande émis par le programme exécuté sur le premier terminal mobile (3.1) sont transmis à l'unité de calcul (2) ; et
- l'unité de calcul (2) du véhicule commande l'au moins un actionneur (1) disposé dans le véhicule pour la génération du retour haptique en fonction des signaux de commande reçus du premier terminal mobile (3.1) ;
dans lequel
le programme exécuté sur le premier terminal mobile (3.1) déclenche l'émission de signaux de commande en fonction d'instructions permettant d'effectuer des exercices de respiration, dans lequel
plusieurs passagers effectuent simultanément un entraînement respiratoire, dans lequel l'exercice de respiration est adapté individuellement à chacun des passagers, de sorte que chaque passager suive un entraînement individuel.

2. Procédé selon la revendication 1 ou 2,
dans lequel
le retour haptique émis dans le véhicule est adapté, en termes de modulation, par rapport au retour haptique prévu pour être émis par l'intermédiaire du premier terminal mobile (3.1).

3. Procédé selon la revendication 1 ou 2,
dans lequel
en plus du retour haptique émis dans le véhicule, des informations dépendant du contexte des signaux de commande reçus du premier terminal mobile (3.1) sont affichées sur un écran (5) dans le véhicule.

4. Procédé selon l'une des revendications 1 à 3,
dans lequel
le passager règle la manière dont le retour haptique est émis dans le véhicule.

5. Procédé selon la revendication 3 ou selon les revendications 3 et 4,
dans lequel
le passager règle la manière dont les informations sont affichées à l'écran dans le véhicule.

6. Procédé selon l'une des revendications 1 à 5,
dans lequel
lorsque l'unité de calcul (2) du véhicule reçoit des signaux de commande provenant du premier terminal mobile (3.1), l'unité de calcul (2) envoie au passager une invitation à effectuer une action de confirmation et n'émet le retour haptique dans le véhicule qu'une fois que le passager a effectué l'action de confirmation.

7. Véhicule comportant une unité de calcul (2) et une interface de communication (6), dans lequel
l'unité de calcul (2) et l'interface de communication (6) sont configurées pour la mise en œuvre d'un procédé selon l'une des revendications 1 à 6.
